# FASCICULE DE BREVET EUROPEEN

(11) **EP 1 976 586 B1**
(45) Date de publication et mention de la délivrance du brevet: **15.03.2017**
(21) Numéro de dépôt: 07718057.8
(22) Date de dépôt: 19.01.2007
(51) Int. Cl.: A61M 25/00, A61L 31/04

(54) **DISPOSITIF MEDICAL INTRALUMINAL CONDITIONNE**
KONDITIONNIERTE INTRALUMINALE MEDIZINISCHE VORRICHTUNG
PACKAGED INTRALUMINAL MEDICAL DEVICE

(30) Priorité: 20.01.2006 FR 0600544
(43) Date de publication de la demande: 08.10.2008
(73) Titulaire: B.Braun Medical SAS, 92100 Boulogne-Billancourt (FR)
(72) Inventeur: BORDEAU, Jérome, F-61110 Condeau (FR); COLLIN, Rémi, F-61340 Saint Hilaire sur Erre (FR)
(74) Mandataire: Ipside
(86) Numéro de dépôt international: PCT/FR2007/000106
(87) Numéro de publication internationale: WO 2007/083033

(56) Documents cités:
- EP-A- 0 372 721
- WO-A-97/47349
- US-A- 3 255 880
- US-A- 5 372 254
- US-A1- 2005 109 648

## Description

La présente invention concerne un dispositif médical intraluminal destiné à être introduit dans un conduit corporel, et, plus précisément un tel dispositif placé dans un conditionnement ayant la forme d'une enveloppe spécifiquement adaptée.

On utilise couramment des dispositifs médicaux intralu-minaux qui comportent un tube mince suffisamment rigide pour ne pas se plier sur lui-même et suffisamment souple pour suivre les courbes d'un conduit corporel, par exemple l'urètre d'un patient, le tube ayant une première extrémité, destinée à être introduite dans le conduit corporel, une seconde extrémité raccordée à un organe récepteur, et une partie d'introduction comprise entre la première extrémité et un emplacement disposé entre les deux extrémités, la partie d'introduction étant destinée à être insérée au moins partiellement dans le conduit corporel. Ces dispositifs sont disposés chacun dans une enveloppe formée par deux feuilles superposées de matière plastique, solidarisées l'une à l'autre, au moins sur des côtés sensiblement parallèles au tube mince, par au moins une bande de liaison de largeur au moins égale à une valeur minimale, et destinée à enfermer de manière étanche, avant utilisation, la partie d'introduction du tube mince, l'enveloppe étant destinée à former une ouverture pour le passage de la première extrémité du tube hors de l'enveloppe afin qu'elle soit introduite dans le conduit corporel, et ayant à cet effet une partie de séparation préférentielle pour la formation de cette ouverture.

La partie de tube mince devant être introduite dans le conduit corporel varie plus ou moins d'un patient à un autre. De plus, comme le tube est en général muni d'un lubrifiant et pour que, après ouverture de l'enveloppe, l'état de stérilité de la partie de tube à introduire soit préservée au maximum, il est souhaitable que seule cette partie soit dégagée de l'enveloppe. En outre, il est souhaitable que l'enveloppe puisse être déchirée à des emplacements différents suivant sa longueur pour dégager l'extrémité à raccorder, pour dégager l'extrémité d'introduction et/ou pour séparer l'enveloppe à un emplacement intermédiaire de sa longueur lorsque l'utilisateur le souhaite.

On connaît donc déjà la formation, sur l'enveloppe, de parties de séparation préférentielle, formées par exemple par amincissement du matériau de l'enveloppe et repérées par un trait transversal. L'utilisateur saisit l'enveloppe de part et d'autre du trait et écarte les mains pour la déchirer. Cette méthode présente des inconvénients car elle nécessite le plus souvent le serrage du tube qui risque ainsi d'être localement dépourvu d'une partie de son lubrifiant, et la manipulation est malaisée, notamment par les personnes dont la dextérité est réduite, et comporte en outre un risque de déplacement du lubrifiant.

On connaît aussi, d'après le document EP-372 721, un conditionnement d'un cathéter muni d'une fibre optique. Ce conditionnement délimite deux compartiments, dont l'un doit rester stérile alors que l'autre est ouvert. Le conditionnement comporte une base qui délimite deux compartiments reliés par une partie rétrécie, et une feuille d'enveloppe en polymère orienté qui couvre la base du dispositif. Une partie de la feuille peut être retirée d'un compartiment, et, à cet effet, elle peut être séparée préférentiellement le long d'une ligne disposée entre les deux compartiments. L'ouverture se fait par décollage de la feuille fixée à la base du compartiment, et non par déchirure de la feuille et de la base.

L'invention a pour objet la réalisation d'un dispositif médical intraluminal, muni d'une enveloppe de conditionnement formées de deux feuilles superposées, dans lequel la séparation préférentielle des deux feuilles à un ou plusieurs emplacements choisis de l'enveloppe peut être réalisée d'une manière très commode.

Plus précisément, l'invention met en oeuvre plusieurs moyens qui, séparément ou en combinaison, facilitent l'utilisation de tels dispositifs médicaux intraluminaux. Ces moyens sont d'une part l'utilisation d'un matériau particulier, constitué d'un polymère orienté, pour la formation des deux feuilles de l'enveloppe, et d'autre part la formation de parties spécifiques de préhension, notamment la disposition spécifique des parties de préhension et des parties de séparation préférentielle.

Ainsi, selon l'invention, l'application d'une force entre une zone de saisie et une partie de préhension provoque une séparation à un emplacement déterminé. L'obtention d'une séparation à un autre emplacement nécessite la saisie d'une zone de saisie et d'une autre partie de préhension.

Plus précisément, l'invention concerne un dispositif médical intraluminal destiné à être introduit dans un conduit corporel, comprenant un tube mince suffisamment rigide pour ne pas se plier sur lui-même et suffisamment souple pour suivre les courbes d'un conduit corporel, le tube ayant une première extrémité, destinée à être introduite dans le conduit corporel, une seconde extrémité raccordée à un organe récepteur, et une partie d'introduction comprise entre la première extrémité et un emplacement disposé entre les deux extrémités, la partie d'introduction étant destinée à être insérée au moins partiellement dans le conduit corporel, et une enveloppe formée par deux feuilles superposées de matière plastique, solidarisées l'une à l'autre, au moins sur des côtés sensiblement parallèles au tube mince, par au moins une bande de liaison de largeur au moins égale à une valeur minimale, et destinée à enfermer de manière étanche, avant utilisation, la partie d'introduction du tube mince, l'enveloppe étant destinée à former une ouverture pour le passage de la première extrémité du tube hors de l'enveloppe pour son introduction dans le conduit corporel, et ayant à cet effet au moins une partie de séparation préférentielle pour la formation d'une ouverture ; selon l'invention, la matière plastique des feuilles de l'enveloppe est un polymère orienté, et la direction d'orientation du polymère orienté est sensiblement transversale à la direction d'allongement du tube du dispositif intraluminal.

De préférence, la partie de séparation préférentielle comporte, dans une bande de liaison au moins, une encoche qui réduit la largeur de la bande de liaison et forme une amorce pour la déchirure des deux feuilles de matière plastique.

Dans un mode de réalisation avantageux, les feuilles superposées de l'enveloppe forment au moins une partie de préhension et une zone de saisie, la partie de préhension et la zone de saisie étant disposées de part et d'autre de la partie de séparation préférentielle.

Dans une variante, l'enveloppe comporte au moins deux parties de séparation préférentielle et au moins deux parties de préhension dont l'une est disposée entre les deux parties de séparation préférentielle et l'autre en dehors du segment d'enveloppe compris entre les deux parties de séparation préférentielle.

Il est avantageux qu'une partie de préhension au moins soit décalée latéralement vers l'extérieur le long de l'enveloppe. Dans un exemple, l'enveloppe comporte deux parties de préhension d'extrémité et une partie intermédiaire de préhension décalée latéralement vers l'extérieur le long de l'enveloppe . Il est alors avantageux qu'une amorce pour la déchirure des deux feuilles de matière plastique soit formée à proximité de la partie intermédiaire de préhension décalée latéralement.

De préférence, la matière plastique des feuilles superposées est choisie parmi le polyéthylène orienté et le polyéthylène bi-orienté.

Dans un mode de réalisation, l'organe récepteur est un embout de fixation à une poche.

Dans un autre mode de réalisation, l'organe récepteur est une poche.

De préférence, le tube mince porte un matériau lubrifiant au moins à la surface de la partie d'introduction.

L'invention concerne aussi l'application d'un dispositif intraluminal tel que défini au sondage urinaire d'un corps humain.

Le document EP-372 721 ne décrit ni ne suggère le fait que la partie de dispositif (cathéter) qui est dégagée est celle qui doit être introduite dans le conduit corporel, ni le fait que l'ouverture par déchirure des feuilles du polymère orienté s'effectue pour ouvrir la cavité délimitée entre les deux feuilles, puisqu'il ne décrit que la séparation d'une feuille collée sur une base.

Dans le présent mémoire, on appelle "zone de saisie" une partie étendue du dispositif qui peut être tenue par pincement entre deux doigts à un emplacement de l'enveloppe qui est normalement comprise à l'intérieur de la bande de liaison. On appelle "partie de préhension" une partie étendue de l'enveloppe qui peut être tenue par pincement entre deux doigts à un emplacement de l'enveloppe qui est normalement à l'extérieur de la bande de liaison et qui est formée par un prolongement des feuilles de l'enveloppe.

Dans le présent mémoire, le terme "étanche" utilisé en référence aux feuilles de matière plastique indique que celles-ci ne laissent pas passer les liquides, bien qu'elles puissent avoir une certaine perméabilité aux gaz, notamment pour permettre la stérilisation.

D'autres caractéristiques et avantages de l'invention seront mieux compris à la lecture de la description qui va suivre d'exemples de réalisation, faite en référence aux dessins annexés sur lesquels :
la figure 1 est une vue schématique d'un dispositif médical intraluminal selon l'invention, permettant la description de caractéristiques de l'invention ;
la figure 2 représente sous forme agrandie une partie de la figure 1 ;
la figure 3 illustre une variante utile pour la réalisation de dispositifs selon l'invention ; et
les figures 4, 5 et 6 représentent trois variantes de réalisation de dispositifs à poche n'ayant que l'un des perfectionnements selon l'invention.

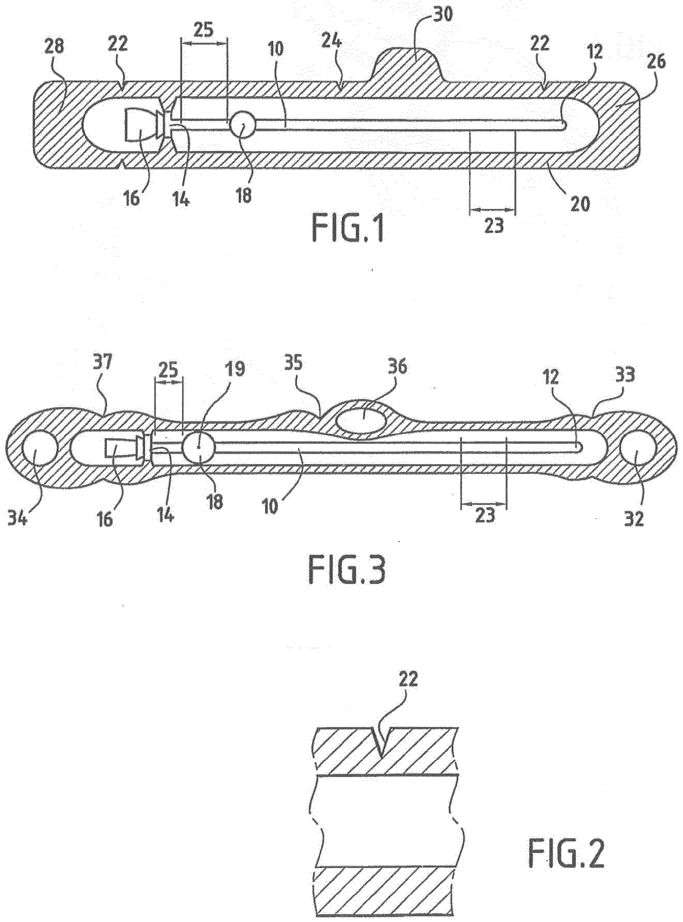

La figure 1 représente un dispositif médical intraluminal selon un mode de réalisation de l'invention qui combine les deux aspects de l'invention. Plus précisément, il comporte un tube 10 destiné à être introduit dans un conduit corporel, tel que l'urètre, et portant à l'extérieur un lubrifiant. La première extrémité 12 du tube est destinée à être introduite dans le conduit corporel, et l'autre extrémité 14 est raccordée à un organe récepteur 16, par exemple un embout de raccordement à une poche. Le tube porte de préférence un système évent 18 destiné à permettre la respiration de l'enveloppe. Cet évent comprend par exemple une perforation 19 adjacente à un organe perméable aux gaz mais pas aux liquides, par exemple une pastille de mousse ou d'un film perméable aux gaz ou d'un non-tissé. Cet organe peut être collé ou soudé à une feuille. Bien qu'on ait représenté ce système évent sur une partie dégagée de la feuille, il peut se trouver au niveau du raccord de l'embout 16 et du tube 10, à proximité de l'extrémité 14.

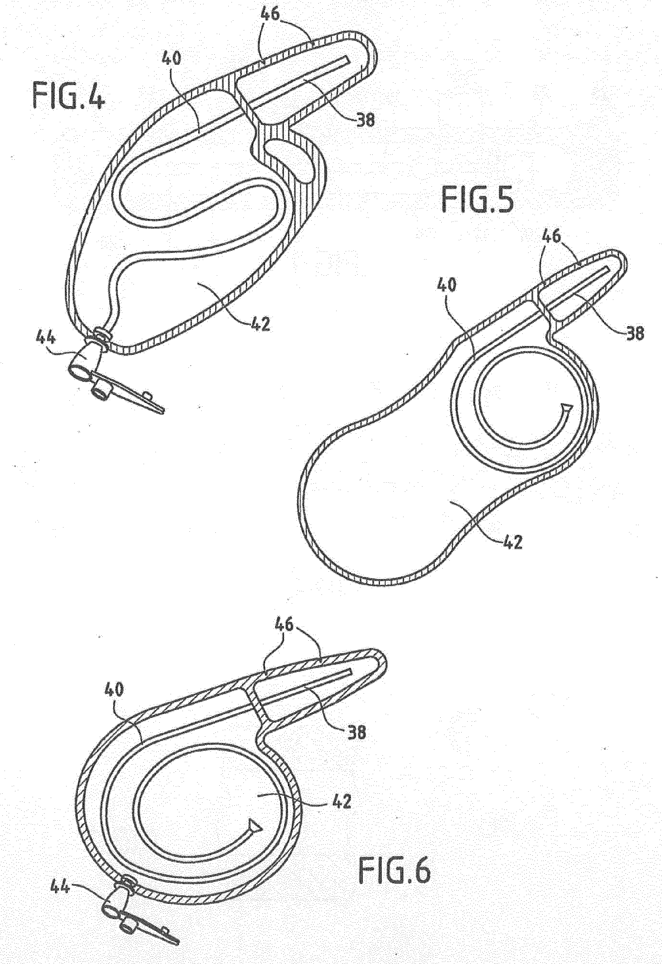

L'organe 16 n'est cependant qu'un exemple, car le tube 10 peut aussi être directement raccordé à une poche réceptrice, comme suggéré par les figures 5 et 6.

Dans le mode de réalisation de la figure 1, une enveloppe est constituée par deux feuilles de matière plastique soudées à la périphérie de l'enveloppe afin que le tube 10 se trouve enfermé dans une cavité.

Bien entendu, suivant les techniques courantes, les feuilles de matière plastique utilisées peuvent avoir une certaine perméabilité afin qu'elles permettent la stérilisation du tube, selon les techniques classiques, par exemple par utilisation de vide et d'oxyde d'éthylène.

Les bandes de liaison latérale de l'enveloppe ont des encoches telles que 22, 24 pour amorcer une déchirure, ayant par exemple la forme d'un V ou d'une simple incision. Selon la technique connue, au niveau de la séparation préférentielle voulue, les feuilles de matière plastique peuvent avoir une épaisseur réduite qui facilite ainsi la déchirure.

Une première caractéristique avantageuse de l'invention est la formation des feuilles de matière plastique en un polymère orienté dont la direction d'orientation est transversale à la longueur du tube 10 et de l'enveloppe représentés sur la figure 1, si bien que l'enveloppe se coupe facilement en direction transversale. A cet effet, le polymère orienté, est de préférence une polyoléfine, avantageusement du polyéthylène orienté ou bi-orienté.

Une seconde caractéristique de l'invention est l'utilisation de parties de préhension telles que 26, 28 et 30. Ces parties sont formées par des parties étendues des feuilles de matière plastique, permettant une saisie ferme par pincement entre deux doigts.

Dans le mode de réalisation de la figure 1, lorsque le dispositif est saisi entre deux doigts d'une main par la partie de préhension 26 et entre deux doigts de l'autre main dans la zone de saisie 23 et lorsqu'une force d'écartement est appliquée, l'enveloppe se déchire transversalement depuis l'encoche 22. Lorsque le dispositif est saisi entre deux doigts d'une main par la partie de préhension 28 et entre deux doigts de l'autre main dans la zone de saisie 25 et lorsqu'une force d'écartement est appliquée, l'enveloppe se déchire transversalement depuis l'autre encoche 22. Lorsque le dispositif est saisi entre deux doigts d'une main par la partie de préhension 30 et entre deux doigts de l'autre main dans la zone de saisie 25, et lorsqu'une force d'écartement est appliquée, l'enveloppe se déchire transversalement depuis l'encoche 24 d'autant plus facilement que l'axe d'application de la force n'est pas confondu avec l'axe longitudinal et la force se concentre sur l'encoche 24.

Cette sélection de la partie de séparation préférentielle par simple sélection des zones de saisie et des parties de préhension est une première caractéristique avantageuse de l'invention.

La figure 3 représente un autre mode de réalisation dans lequel les parties de préhension comportent des orifices tels que 32, 34, 36 permettant le passage d'un doigt. De cette manière, la force appliquée peut être très élevée et la manipulation est très simple. Suivant l'orifice dans lequel passe un doigt d'une main et la zone de saisie par deux doigts de l'autre main, l'enveloppe se sépare à des emplacements différents, indiqués par les encoches 33, 35, 37.

Bien entendu, le nombre de parties de préhension, comprenant des pattes telles que 30 ou des orifices tels que 32, 36, n'est pas limité. Cependant, un plus grand nombre risque de paraître compliqué à l'utilisateur, qui n'a par contre guère de problème pour comprendre l'utilisation de trois parties de préhension réparties sur la longueur de l'enveloppe.

Les figures 4 à 6 représentent trois modes de réalisation dans lesquels le tube ou sonde 38 est relié à un tube souple 40 qui est disposé dans une poche 42. La poche peut comporter un bouchon de sortie 44 et le tube 40 peut être relié à celui-ci, comme indiqué sur la figure 4. Le tube peut aussi être simplement enroulé sur lui-même comme indiqué sur les figures 5 et 6.

Selon l'invention, les feuilles de ces poches sont formées de polymère orienté tel qu'une déchirure qui s'amorce à une encoche 46 traverse la partie antérieure du dispositif qui contient la sonde 38 et permet l'accès à celle-ci.

La matière plastique des feuilles superposées est avantageusement choisie parmi les polyoléfines, telles que le polyéthylène orienté dans une seule direction ou dans plusieurs directions ; lorsqu'il est orienté dans plusieurs directions, les coefficients d'étirage, et donc d'orientation, ne sont pas les mêmes dans les différentes directions et il existe un effet global résultant d'orientation préférentielle, de préférence alignée suivant la largeur de l'enveloppe.

## Revendications

1. Dispositif médical intraluminal destiné à être introduit dans un conduit corporel, comprenant un tube mince (10) suffisamment rigide pour ne pas se plier sur lui-même et suffisamment souple pour suivre les courbes d'un conduit corporel,
le tube (10) ayant une première extrémité (12), destinée à être introduite dans le conduit corporel, une seconde extrémité (14) raccordée à un organe récepteur (16), et une partie d'introduction comprise entre la première extrémité et un emplacement disposé entre les deux extrémités, la partie d'introduction étant destinée à être insérée au moins partiellement dans le conduit corporel, et
une enveloppe (20) formée par deux feuilles superposées de matière plastique, solidarisées l'une à l'autre, au moins sur des côtés sensiblement parallèles au tube mince (10), par au moins une bande de liaison de largeur au moins égale à une valeur minimale, et destinée à enfermer de manière étanche, avant utilisation, la partie d'introduction du tube mince (10),
l'enveloppe (20) étant destinée à former une ouverture pour le passage de la première extrémité (12) du tube hors de l'enveloppe (20) pour son introduction dans le conduit corporel, et ayant à cet effet au moins une partie de séparation préférentielle pour la formation d'une ouverture,
la matière plastique des feuilles de l'enveloppe (20) étant un polymère orienté, et la direction d'orientation du polymère orienté étant sensiblement transversale à la direction d'allongement du tube du dispositif intraluminal,
**caractérisé**
**en ce que** les feuilles superposées de l'enveloppe (20) forment au moins deux parties de préhension d'extrémité (26, 32, 28, 34), une partie intermédiaire de préhension (30, 36) décalée latéralement vers l'extérieur le long de l'enveloppe (20) et une zone de saisie (23, 25),
et **en ce que** au moins une partie de préhension et une zone de saisie sont disposées de part et d'autre d'une partie de séparation préférentielle

2. Dispositif intraluminal selon la revendication 1, **caractérisé en ce que** la partie de séparation préférentielle (22, 24 ; 33, 35, 37, 46) comporte, dans une bande de liaison au moins, une encoche qui réduit la largeur de la bande de liaison et forme une amorce pour la déchirure des deux feuilles de matière plastique.

3. Dispositif intraluminal selon l'une quelconque des revendications précédentes, **caractérisé en ce que** l'enveloppe (20) comporte au moins deux parties de séparation préférentielle (22, 24 ; 33, 35, 37, 39) et au moins deux parties de préhension (26, 28, 30 ; 32, 34, 36) dont l'une (30 ; 36) est disposée entre les deux parties de séparation préférentielle et l'autre en dehors du segment d'enveloppe compris entre les deux parties de séparation préférentielle.

4. Dispositif intraluminal selon l'une l'une quelconque des revendications précédentes, **caractérisé en ce qu'**une partie de préhension (30 ; 36) au moins est décalée latéralement vers l'extérieur le long de l'enveloppe (20).

5. Dispositif intraluminal l'une quelconque des revendications précédentes, **caractérisé en ce qu'**une amorce pour la déchirure des deux feuilles de matière plastique est formée à proximité de la partie intermédiaire de préhension (36) décalée latéralement.

6. Dispositif intraluminal selon l'une quelconque des revendications précédentes, **caractérisé en ce que** la matière plastique des feuilles superposées est choisie parmi le polyéthylène orienté et le polyéthylène biorienté.

7. Dispositif intraluminal selon l'une quelconque des revendications précédentes, **caractérisé en ce que** l'organe récepteur est un embout (16) de fixation à une poche.

8. Dispositif intraluminal selon l'une quelconque des revendications 1 à 6, **caractérisé en ce que** l'organe récepteur est une poche

## Patentansprüche

1. Intraluminale medizinische Vorrichtung, die dazu bestimmt ist, in einen Körperkanal eingeführt zu werden, umfassend eine dünne Röhre (10), die starr genug ist, um sich nicht auf sich selbst umzubiegen, und biegsam genug ist, um den Kurven eines Körperkanals zu folgen,
wobei die Röhre (10) ein erstes Ende (12), das dazu bestimmt ist, in den Körperkanal eingeführt zu werden, und ein zweites Ende (14), das an ein Aufnahmeelement (16) angeschlossen ist, und einen Einführteil, der zwischen dem ersten Ende und einer Stelle zwischen den beiden Enden enthalten ist, umfasst, wobei der Einführteil dazu bestimmt ist, zumindest teilweise in den Körperkanal eingesetzt zu werden, und
eine Hülle (20), die von zwei übereinander liegenden Folien aus Kunststoff gebildet ist, die miteinander zumindest auf im Wesentlichen zur dünnen Röhre (10) parallelen Seiten durch mindestens ein Verbindungsband mit einer Breite zumindest gleich einem Mindestwert, das dazu bestimmt ist, den Einführteil der dünnen Röhre (10) vor der Verwendung dicht einzuschließen, verbunden sind,
wobei die Hülle (20) dazu bestimmt ist, eine Öffnung für den Durchgang des ersten Endes (12) der Röhre aus der Hülle (20) für seine Einführung in den Körperkanal zu bilden, wobei sie zu diesem Zweck mindestens einen bevorzugten Abtrennungsteil zur Bildung einer Öffnung aufweist,
wobei der Kunststoff der Folien der Hülle (20) ein orientiertes Polymer ist, und die Orientierungsrichtung des orientierten Polymers im Wesentlichen quer zur Erstreckungsrichtung der Röhre der intraluminalen Vorrichtung ist,
**dadurch gekennzeichnet, dass** die übereinander liegenden Folien der Hülle (20) mindestens zwei Endgreifteile (26, 32, 28, 34), einen Zwischengreifteil (30, 36), der seitlich nach außen entlang der Hülle (20) versetzt ist, und eine Greifzone (23, 25) bilden,
und dass mindestens ein Greifteil und eine Greifzone beiderseits eines bevorzugten Abtrennungsteils angeordnet sind.

2. Intraluminale Vorrichtung nach Anspruch 1, **dadurch gekennzeichnet, dass** der bevorzugte Abtrennungsteil (22, 24; 33, 35, 37, 46) mindestens in einem Verbindungsband eine Kerbe umfasst, die die Breite des Verbindungsbandes reduziert und einen Ansatz zum Zerreißen der beiden Kunststofffolien bildet.

3. Intraluminale Vorrichtung nach einem der vorhergehenden Ansprüche, **dadurch gekennzeichnet, dass** die Hülle (20) mindestens zwei bevorzugte Abtrennungsteile (22, 24; 33, 35, 37, 39) und mindestens zwei Greifteile (26, 28, 30; 32, 34, 36) umfasst, von denen einer (30; 36) zwischen den beiden bevorzugten Abtrennungsteilen und der andere außerhalb des Hüllensegments, das zwischen den beiden bevorzugten Abtrennungsteilen enthalten ist, angeordnet ist.

4. Intraluminale Vorrichtung nach einem der vorhergehenden Ansprüche, **dadurch gekennzeichnet, dass** mindestens ein Greifteil (30; 36) seitlich nach außen entlang der Hülle (20) versetzt ist.

5. Intraluminale Vorrichtung nach einem der vorhergehenden Ansprüche, **dadurch gekennzeichnet, dass** ein Ansatz zum Zerreißen der beiden Kunststofffolien in der Nähe des seitlich versetzten Zwischengreifteils (36) ausgebildet ist.

6. Intraluminale Vorrichtung nach einem der vorhergehenden Ansprüche, **dadurch gekennzeichnet, dass** der Kunststoff der übereinander liegenden Folien unter dem orientierten Polyethylen und dem bi-orientierten Polyethylen ausgewählt ist.

7. Intraluminale Vorrichtung nach einem der vorhergehenden Ansprüche, **dadurch gekennzeichnet, dass** das Aufnahmeelement ein Ansatz (16) zur Befestigung an einer Tasche ist.

8. Intraluminale Vorrichtung nach einem der Ansprüche 1 bis 6, **dadurch gekennzeichnet, dass** das Aufnahmeelement eine Tasche ist.

## Claims

1. Intraluminal medical device intended to be inserted into a bodily duct, comprising a thin tube (10) that is rigid enough not to fold up on itself and flexible enough to follow the curves of a bodily duct,
the tube (10) having a first end (12), intended to be inserted into the bodily duct, a second end (14), connected to a receiving element (16), and an insertion part situated between the first end and a site located between the two ends, the insertion part being intended to be inserted at least partially into the bodily duct, and
an envelope (20) formed by two superposed sheets of plastic material which are rigidly connected to each other, at least on sides substantially parallel to the thin tube (10), by at least one connecting strip having a width at least equal to a minimum value and being intended to enclose the insertion part of the thin tube (10) in a sealed manner before use,
the envelope (20) being intended to form an opening for the passage of the first end (12) of the tube out of the envelope (20) for its insertion into the bodily duct, and having for this purpose at least one preferred separation part for the formation of an opening,
the plastic material of the sheets of the envelope (20) being an oriented polymer, and the direction of orientation of the oriented polymer being substantially transverse to the direction of elongation of the tube of the intraluminal device, **characterized in that** the superposed sheets of the envelope (20) form at least two end gripping parts (26, 32, 28, 34), an intermediate gripping part (30, 36) offset laterally towards the outside along the envelope (20), and a holding zone (23, 25),
and **in that** at least one gripping part and one holding zone are arranged on either side of a preferred separation part.

2. Intraluminal device according to Claim 1, **characterized in that** the preferred separation part (22, 24; 33, 35, 37, 46) has, in at least one connecting strip, a notch that reduces the width of the connecting strip and forms an initiator for tearing the two sheets of plastic material.

3. Intraluminal device according to either of the preceding claims, **characterized in that** the envelope (20) has at least two preferred separation parts (22, 24; 33, 35, 37, 39) and at least two gripping parts (26, 28, 30; 32, 34, 36), of which one (30; 36) is arranged between the two preferred separation parts and the other is arranged outside the envelope segment located between the two preferred separation parts.

4. Intraluminal device according to any one of the preceding claims, **characterized in that** one gripping part (30; 36) at least is offset laterally towards the outside along the envelope (20).

5. Intraluminal device according to any one of the preceding claims, **characterized in that** an initiator for tearing the two sheets of plastic material is formed near the intermediate gripping part (36) that is offset laterally.

6. Intraluminal device according to any one of the preceding claims, **characterized in that** the plastic material of the superposed sheets is chosen from between oriented polyethylene and bi-oriented polyethylene.

7. Intraluminal device according to any one of the preceding claims, **characterized in that** the receiving element is a nozzle (16) for attachment to a pouch.

8. Intraluminal device according to any one of Claims 1 to 6, **characterized in that** the receiving element is a pouch.
